# EUROPEAN PATENT SPECIFICATION

(11) **EP 4 540 346 B1**
(45) Date of publication and mention of the grant of the patent: **11.02.2026**
(21) Application number: 23727292.7
(22) Date of filing: 15.05.2023
(51) Int. Cl.: C10G 47/16, C10G 47/18

(54) **PROCESS FOR ACTIVATION OF A HYDROGENOLYSIS CATALYST**
VERFAHREN ZUR AKTIVIERUNG EINES HYDROGENOLYSEKATALYSATORS
PROCÉDÉ D'ACTIVATION D'UN CATALYSEUR D'HYDROGÉNOLYSE

(30) Priority: 14.06.2022 EP 22179046
(43) Date of publication of application: 23.04.2025
(73) Proprietor: SABIC Global Technologies B.V., 4612 PX Bergen op Zoom (NL)
(72) Inventor: DASARI, Prasanna R., Bengaluru 562125 (IN); FICKEL, Dustin, Bengaluru 562125 (IN); BARTON, Katherine, Bengaluru 562125 (IN)
(74) Representative: J A Kemp LLP
(86) International application number: PCT/EP2023/062994
(87) International publication number: WO 2023/241872

(56) References cited:
- CA-A- 1 196 623
- US-A- 2 853 435
- US-A- 3 998 755
- MORRIS ARGYLE ET AL: "Heterogeneous Catalyst Deactivation and Regeneration: A Review", CATALYSTS, vol. 5, no. 1, 26 February 2015 (2015-02-26), pages 145 - 269, XP055652473, DOI: 10.3390/catal5010145

## Description

### BACKGROUND OF THE INVENTION

### A. Field of the Invention

The invention generally concerns processes for activation of catalysts for hydrocarbon hydrogenolysis. The process includes contacting an oxidized catalyst with a stream comprising butane in the presence of H₂ to form a treated catalyst. The treated catalyst is then contacted with H₂ to form an activated hydrogenolysis catalyst. The source of the oxidized catalyst can be a fresh catalyst or deactivated catalyst that has been exposed to, for example, oxygen.

### B. Description of Related Art

Light alkanes, particularly ethylene, can be obtained through a hydrocarbon (e.g., butane (C4)) steam cracking process. However, steam cracking of a hydrocarbon feedstock can produce a relatively low yield of ethylene. A combination of hydrocarbon hydrogenolysis and steam cracking selectively can produce desired olefins. In some instances, hydrogenolysis reaction can be advantageous.

In the hydrogenolysis of hydrocarbon reaction it is desirable for hydrocarbon hydrogenolysis catalysts to have selectivity toward ethane to minimize the carbon loss to byproducts (e.g., methane and propane). Conventional iridium (Ir) or IrPt based catalysts can have an ethane selectivity of 60 - 70% at a hydrocarbon conversion level of about 80 - 90%. However, the use of noble metals such as Ir and platinum (Pt) can result in significant capital cost. For example, Ir is not readily available and can be difficult to recover from a spent catalyst.

To lower operating costs, hydrogenolysis catalysts can be regenerated. Conventional catalyst regeneration strategies in hydrocarbon processes predominantly involve burning of carbon deposits (causing deactivation) in an oxidative atmosphere. However depending on the sensitivity of the active site to the oxidation temperature, this step is replaced or followed by an oxychlorination step in order to maintain the active site dispersion, which is both complex and capital intensive. For example, U.S. Patent No. 3998755 to John Hayes titled "Regeneration of a coke-deactivated, acidic bimetallic Pt-Ir catalyst and Canadian Patent No. 1196623 to Weissman *et.al.,* titled "Process for reactivating iridium-containing catalysts" both describe contacting spent catalysts at an elevated temperature in the presence of oxygen, water, and halides.

While attempts to reactivate hydrogenolysis catalysts have been described, there is still a need for cost effective processes. Therefore, the following provides a solution to the problem of the need for improvement in methods to activate hydrogenolysis catalysts, which is relatively simple and cost-effective to implement in a manufacturing operation in comparison to existing methods.

US3998755 discloses reactivation of a catalyst comprising platinum and/or palladium and further iridium as active metal.

### SUMMARY OF THE INVENTION

A solution to at least one problem associated with activation of hydrogenolysis catalysts has been discovered. At least one solution can include contacting an oxidized catalyst with a hydrocarbon containing stream in the presence of hydrogen (H₂) to form a hydrocarbon treated catalyst. The oxidized catalyst can be a fresh or deactivated catalyst that has been treated or exposed to oxidizing conditions (e.g., exposure to oxygen (O₂)). The hydrocarbon treated catalyst can then be contacted with H₂ to form an activated hydrogenolysis catalyst. The H₂ used to form the hydrocarbon treated catalyst and the H₂ used to form the activated hydrogenolysis catalyst can be from the same H₂ source or stream. The H₂ used to form the hydrocarbon treated catalyst can be from a first H₂ source or stream, and the H₂ used to form the activated hydrogenolysis catalyst can be from a second H₂ source or stream. Activating the catalyst in such a manner can increase the hydrocarbon conversion stability of the catalyst as compared to hydrogenolysis of hydrocarbons with the same catalyst that has not been activated in a manner of the present invention. Activation can include regeneration of a spent catalyst, activation of a mixture of spent and fresh catalyst during a hydrogenolysis reaction (e.g., after some hydroconversion has dropped), or a pretreatment of a fresh hydrogenolysis catalyst. A benefit of the processes of the present invention can include activation of fresh and/or spent catalysts in a cost efficient manner. Still further, it is believed that the processes of the present invention can modify the active site structure of the catalysts thereby making the catalysts more stable over time, which can further extend the life of the catalyst and delay the need to replace or regenerate spent catalysts.

The present invention provides a process for the activation of a hydrogenolysis catalyst the process comprising: (a) contacting an oxidized catalyst with a stream comprising butane in the presence of H₂ to form a treated catalyst, and thereafter (b) contacting the treated catalyst of step (a) with H₂ to form an activated hydrogenolysis catalyst. The stream comprising butane may comprise n-butane, isobutane or a combination thereof. The stream comprising butane of step (a) can include further C2 to C10 hydrocarbons, preferably C2 to C5 hydrocarbons. In some aspects, a weight ratio of H₂ to hydrocarbon(s) in step (a) can be 0.5 to 20. In other aspects, a weight ratio of hydrocarbon(s) to catalyst can be 1 to 100. Conditions of step (a) can include a temperature of 200 °C to 350 °C, preferably 275 °C to 325 °C, and a pressure of 0.5 MPa to 1.5 MPa, preferably 0.7 MPa to 0.8 MPa. Step (b) can be performed for a time period longer than step (a) and can include a temperature of 300 °C to 500 °C, preferably 300 °C to 400 °C, and a pressure of 0.5 MPa to 1.5 MPa, preferably, 0.7 MPa to 0.8 MPa. The oxidized catalyst of step (a) can be produced by contacting a fresh catalyst, a deactivated catalyst, or mixture thereof with an oxidizing stream that can include oxygen (O₂) and a diluent at a temperature of 200 °C to 450 °C, preferably 300 °C to 400 °C. The oxidizing stream can include 0.1 to 30 vol.% of O₂, preferably 5 to 20 vol.% O₂, or more preferably 8 to 12 vol. % O₂. In some aspects, the catalyst can include at least two noble metals on a support. For example, a fresh hydrogenolysis catalyst containing noble metals can be pretreated by heating the catalyst to the desired temperature and then contacting the heated fresh hydrogenolysis catalyst with the diluted oxygen stream to form the oxidized catalyst of step (a), and then step (b) of the activation process of the present invention can be performed. In another example, a deactivated catalyst (e.g., less than 20% hydrocarbon conversion, more preferably less than 15% hydrocarbon conversion, or 1 to 20% hydrocarbon conversion, or 1%, 5%, 10%, 15%, 20%, or any range or value there between) can be heated to the desired temperature and then contacted with the diluted oxygen stream to form the oxidized catalyst of step (a), and then step (b) of the activation process of the present invention can be performed. In yet another example, during a hydrocarbon hydrogenolysis process, when the catalyst (mixture of fresh and deactivated) has lost some activity (e.g., a drop greater than 20% hydrocarbon conversion, more preferably greater than 20% hydrocarbon conversion, or 21 to 50% hydrocarbon conversion, or 21%, 25%, 30%, 35%, 40%, 45%, 50%, or any range or value there between), the feeds can be stopped, the reaction purged with nitrogen, then the catalyst mixture can be contacted with the diluted oxygen stream to form the oxidized catalyst of step (a), and then step (b) of the activation process of the present invention can be performed. Non-limiting examples of noble metals include platinum (Pt), iridium (Ir), palladium (Pd), ruthenium (Rh), silver (Ag), gold (Au), an alloy thereof, or a combination thereof, preferably PtIr. The catalyst support can be zeolite support, preferably ZSM5.

A method to produce ethane can include contacting the activated hydrogenolysis catalyst produced according to the method of the present invention with a hydrocarbon(s) (e.g., C3-C10 hydrocarbons, preferably, C3 to C5 hydrocarbons, more preferably n-butane, isobutane, or a combination thereof) under conditions sufficient for hydrogenolysis of the hydrocarbon(s) to produce ethane. Advantageously, the hydrocarbon conversion stability is increased as compared to hydrogenolysis of hydrocarbons with the original untreated catalyst under the same hydrogenolysis conditions. Without wishing to be bound by theory, it is believed that the process of the present invention modifies the active site structure and makes the catalyst more stable. In instances where the catalyst is a spent catalyst, catalyst activity is recovered. Hydrogenolysis contacting conditions can include a temperature of 240 °C to 350 °C, preferably 260 °C to 300 °C, a pressure of 0.35 MPa to 1.4 MPa, a weight hourly space velocity (hydrocarbon based WHSV) of 1 to 10 hr⁻¹, preferably 1 to 4 hr⁻¹, or combinations thereof.

The following includes definitions of various terms and phrases used throughout this specification.

The terms "about" or "approximately" are defined as being close to as understood by one of ordinary skill in the art. In one non-limiting embodiment, the terms are defined to be within 10%, preferably within 5%, more preferably within 1%, and most preferably within 0.5%.

The terms "wt.%," "vol.%," or "mol.%" refers to a weight percentage of a component, a volume percentage of a component, or molar percentage of a component, respectively, based on the total weight, the total volume of material, or total moles, that includes the component. In a non-limiting example, 10 grams of component in 100 grams of the material is 10 wt.% of component.

The term "substantially" and its variations are defined to include ranges within 10%, within 5%, within 1%, or within 0.5%.

The terms "inhibiting" or "reducing" or "preventing" or "avoiding" or any variation of these terms, when used in the claims and/or the specification includes any measurable decrease or complete inhibition to achieve a desired result.

The term "effective," as that term is used in the specification and/or claims, means adequate to accomplish a desired, expected, or intended result.

The use of the words "a" or "an" when used in conjunction with any of the terms "comprising," "including," "containing," or "having" in the claims, or the specification, may mean "one," but it is also consistent with the meaning of "one or more," "at least one," and "one or more than one."

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The processes of the present invention can "comprise," "consist essentially of," or "consist of" particular ingredients, components, compositions, *etc.* disclosed throughout the specification. With respect to the transitional phrase "consisting essentially of," in one non-limiting aspect, a basic and novel characteristic of the processes of the present invention are their abilities to activate hydrocarbon hydrogenolysis catalysts. In certain aspects, the processes of the present invention can be performed without using conventional catalyst regeneration strategies such as burning of carbon deposits and/or use of halides (e.g., an oxychlorination step).

Other objects, features and advantages of the present invention will become apparent from the following figures, detailed description, and examples. It should be understood, however, that the figures, detailed description, and examples, while indicating specific embodiments of the invention, are given by way of illustration only.

### BRIEF DESCRIPTION OF THE DRAWINGS

Advantages of the present invention may become apparent to those skilled in the art with the benefit of the following detailed description and upon reference to the accompanying drawings.
**FIG. 1** is an illustration of the activation protocol of the present invention.
**FIG. 2** is an illustration of a reactor system and process to produce ethane using the activated hydrocarbon hydrogenolysis catalyst.
**FIG. 3** is an illustration of conversion of n-butane and product selectivity to ethane as a function of time on stream before and after regeneration (275 °C, H₂/C₄H₁₀ molar ratio = 2.5, weight hourly space velocity w.r.t. C₄H₁₀ = 4 h⁻¹, 0.79 MPa (100 psig)).
FIG. 4 is an illustration of conversion of n-butane as a function of time on stream before and after regeneration without hydrocarbon injection (275 °C, H₂/C₄H₁₀ molar ratio = 2.5, weight hourly space velocity w.r.t. C₄H₁₀ = 4 h⁻¹, 0.79MPa (100 psig)).
**FIG. 5** is an illustration of conversion of n-butane and product selectivity to ethane as a function of time on stream before and after regeneration (275 °C, H₂/C₄H₁₀ molar ratio = 2.5, weight hourly space velocity (WHSV) of C₄H₁₀ = 4 h⁻¹, 100 psig (0.79 MPa)

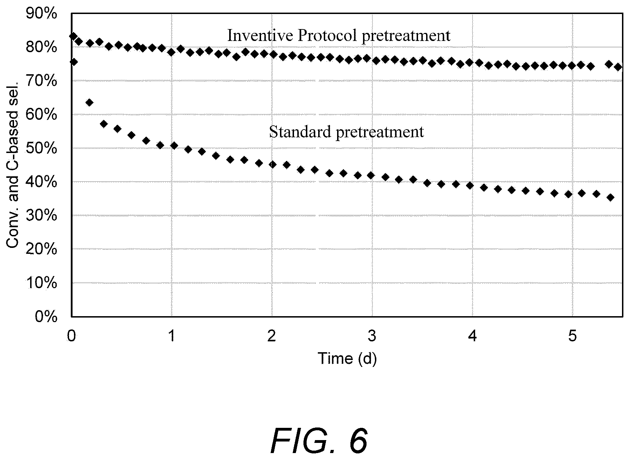

**FIG. 6** is an illustration of comparison of n-butane conversion using two portions of the same catalyst. The top line (inventive protocol pretreatment) shows n-butane conversion for one catalyst portion that was subjected to the inventive treatment protocol pretreatment. The bottom line (standard pretreatment) shows n-butane conversion for the other catalyst portion that was subjected to standard catalyst pretreatment that involved only H₂ reduction at 400 °C. Reaction parameters for both n-butanes conversion were: hydrocarbon WHSV: 4h⁻¹, T: 280 °C, P: 100 psig (0.79 MPa), and the H₂/HC for modified pretreatment was 1.5 and H₂/HC for the standard pretreatment was 2.5.

While the invention is susceptible to various modifications and alternative forms, specific embodiments thereof are shown by way of example in the drawings. The drawings may not be to scale.

### DETAILED DESCRIPTION OF THE INVENTION

While attempts to reactivate hydrogenolysis catalysts have been described, there is still a need for cost effective processes. Therefore, the following provides a solution to the problem of the need for improvement in methods to activate hydrogenolysis catalysts, which is relatively simple and cost-effective to implement in a manufacturing operation in comparison to existing methods.

At least one solution to the problems associated with catalyst activity in the conversion of hydrocarbon to ethane has been discovered. The solution can include a cost-effective activation process of a hydrogenolysis catalyst. The process can increase the stability of the hydrogenolysis catalysts as compared to the original untreated hydrogenolysis catalyst under the same conditions. The activation can be used as a pretreatment of a fresh catalyst or regeneration of a deactivated catalyst.

### A. Process to Activate a Hydrogenolysis Catalyst

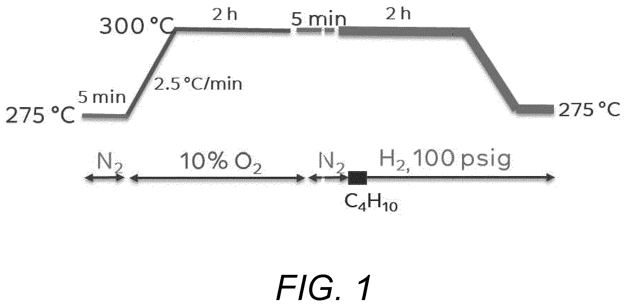

FIG. 1 depicts an illustration of an activation process of the present invention. For example, a hydrogenolysis catalyst can be contacted with nitrogen at an elevated temperature (e.g., 200 °C to 280 °C or 200 °C, 220 °C, 230 °C, 240 °C, 250 °C, 260 °C, 270 °C, 280 °C or any value or range there between) for a period of time (e.g. about 1 to 10 minutes or 1, 2, 3, 4, 5, 6, 7, 8, 9, 10 minutes or any value or range there between). The catalyst can be a fresh catalyst, a deactivated catalyst *(e.g.,* a catalyst that was exposed to the reaction feed for any duration of time and that showed a drop in hydrocarbon conversion activity over the course of the run) or a mixture of fresh and deactivated catalyst (e.g., catalyst losing activity during a hydrogenolysis process).

Next, oxygen can be introduced into a nitrogen stream to form a diluted stream of oxygen (e.g., 0.1 to 30 vol%, or 0.1 vol.%, 1 vol.%, 5 vol.%, 10 vol.%, 15 vol.%, 20 vol.%, 25 vol.%, 30 vol.% or any range or value there between, preferably 5 to 20 vol.%, more preferably 8 to 12 vol.%) and contacted with the heated catalyst at an elevated temperature (e.g., 200 °C to 450 °C, 275 °C to 325 °C, or 200 °C, 225 °C, 250°C, 275 °C, 300°C, 325 °C, 350 °C, 375 °C, 400 °C, or any range or value there between). In some aspects, a pressure during oxygen contact can be 0.5 MPa to 1.5 MPa, preferably 0.7 MPa to 0.8 MPa, or 0.5 MPa, 0.7 MPa, 0.8 MPa, 0.9 MPa, 1.0 MPa, 1.1 MPa, 1.2 MPa, 1.3 MPa, 1.4 MPa, 1.5 MPa or any value or range there between. The temperature can be ramped at a rate of 1 °C to 5 °C per min, or 1 °C, 1.5 °C, 2 °C, 2.5 °C, 3 °C, 3.5 °C, 4 °C, 4.5 °C, or 5 °C or any value or range there between. In one aspect, a ramp rate of 2.5 °C per min can be used. Once the temperature is at a targeted temperature (e.g., 300 °C, 325 °C, 350 °C or any range or value there between), the catalyst can be held in the diluted oxygen stream for a period of time (e.g., 1 to 5 hours or 1, 2, 3, 4, 5 hours, or any range or value there between) to form an oxidized catalyst. Preferably, the heated catalyst is contacted with the diluted oxygen stream for about 2 hours to form the oxidized catalyst.

After the oxidizing step, the oxidized catalyst can then be purged with nitrogen for a period of time *(e.g.* 1 to 5 minutes) to remove loosely bound oxygen and/or any other oxygen within a unit that includes the catalyst *(e.g.,* a reactor). A stream comprising butane and a hydrogen stream can then be contacted with the oxidized catalyst to produce a treated catalyst. The streams can be separate streams or a mixed stream. The oxidized catalyst can be contacted with the stream comprising butane in the presence of H₂ at a desired temperature (e.g., 200 °C to 350 °C, 275 °C to 325 °C, or 200 °C, 225 °C, 250 °C, 275 °C, 300 °C, 325 °C, 350 °C or any range or value there between) to produce the hydrocarbon treated catalyst. A pressure during hydrocarbon/hydrogen contact can be 0.5 MPa to 1.5 MPa, preferably 0.7 MPa to 0.8 MPa, or 0.5 MPa, 0.7 MPa, 0.8 MPa, 0.9 MPa, 1.0 MPa, 1.1 MPa, 1.2 MPa, 1.3 MPa, 1.4 MPa, 1.5 MPa. In some embodiments, a temperature of 275 °C to 325 °C and a pressure of 0.7 MPa to 0.8 MPa is used. Non-limiting examples of optional hydrocarbons in the stream comprising butane include C2 to C10 hydrocarbons, or C2, C3, C4, C5, C6, C7, C8, C9, C10, or any range there between. The butane in the stream comprising butane may be n-butane, isobutane or a combination thereof. In one aspect, a 70/30 vol.% mixture of n-butane/isobutane can be used. A weight ratio of hydrocarbons to catalyst can range from 1 to 100 or 1, 10, 20, 30, 40, 50, 60, 70, 80, 90, 100 or any range or value there between. The hydrogen/hydrocarbon ratio can range between 0.5 to 20 or 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20 or any range or value therebetween. The oxidized catalyst can be contacted with the hydrocarbon stream and H₂ for 20 seconds to 180 minutes, (e.g., 20 sec., 40 sec., 1 min., 5 min., 10 min., 20 min., 30 min., 40 min., 50 min., 60 min., 70 min. 80 min., 90 min., 100 min., 110 min., 120 min., 130 min., 140 min., 150 min., 160 min., 170 min., 180 min., or any value or range there between to produce the hydrocarbon treated catalyst. In one aspect, the catalyst is contacted with the stream comprising butane and H₂ for 2 to 7 min, or 5 min to produce the treated catalyst.

Next, the stream comprising butane can be discontinued and the hydrocarbon treated catalyst can be contacted with hydrogen (99 to 100% purity) to produce the activated catalyst of the present invention. In some embodiments, the amount of hydrocarbon can be decreased over time to increase the amount of H₂ contacting the hydrocarbon treated catalyst. In other aspects, once the treated catalyst is obtained (isolated), then the treated catalyst is contacted with a separate H₂ feed stream. Contact of the treated catalyst with hydrogen can be at a temperature of 300 °C to 500 °C, 300 °C to 400 °C, or 300 °C, 325 °C, 350 °C, 375 °C, 400 °C, 425 °C, 450 °C, or 500 °C or any range or value there between to produce the activated catalyst of the present invention. A pressure during hydrogen contact can be 0.5 MPa to 1.5 MPa, preferably 0.7 MPa to 0.8 MPa, or 0.5 MPa, 0.7 MPa, 0.8 MPa, 0.9 MPa, 1.0 MPa, 1.1 MPa, 1.2 MPa, 1.3 MPa, 1.4 MPa, 1.5 MPa. for 1 to 2 h. The flow of hydrogen and heat can be discontinued to start the cooling process. The activated catalyst can be cooled to about 200 °C to 300 °C, or 250 °C to 275 °C, or 200 °C, 225 °C, 250 °C, 275 °C, 300 °C, or any value or range therebetween. In one aspect, the activated catalyst is cooled to 270 °C to 280 °C, or about 275 °C. In some aspects the combined time for the butane treating step and the activation step can be greater than the time that oxygen is contacted with the catalyst. For example, if the catalyst is contacted with oxygen for 2 hours, the catalyst can then be contacted with a hydrocarbon/ hydrogen mixture for about 5 minutes, and then for 2 hours with substantially pure or pure hydrogen (e.g., 90% or greater, preferably 95% or greater, or more preferably 98 or 99% or greater H₂) to produce the activated catalyst of the present invention.

### B. Methods of Producing Ethane from Hydrocarbons using an Activated Catalyst

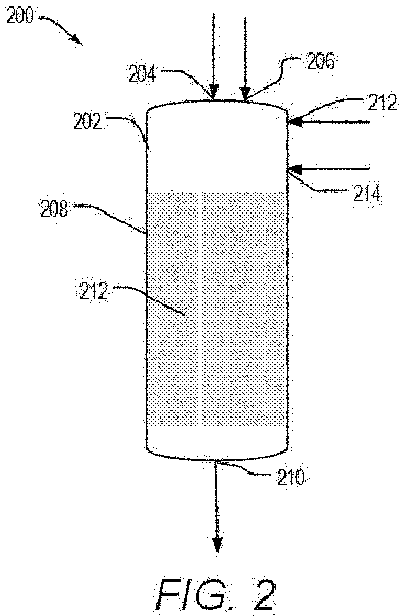

FIG. 2 depicts a schematic for a process for the hydrogenolysis of hydrocarbons with one reactor using the activated catalyst. System 200 can include reactor 202, inlet 204 for a H₂ reactant feed, inlet 206 for a hydrocarbon reactant feed, reaction zone 208 *(e.g.,* a fixed-bed reactor) that is configured to be in fluid communication with the inlets 204 and 206, and outlet 210 configured to be in fluid communication with the reaction zone 208 and configured to remove the hydrogenolysis product stream from the reaction zone. The reaction zone 208 can include activated catalyst 212. Activated catalyst 212 can be a fresh catalyst or a deactivated catalyst that has been placed in reactor 202 and then activated (pretreated or regenerated) using the activation process of the present invention (see above section A) prior to the hydrogenolysis process being started. The activated catalyst may be placed in the reactor after activation. The H₂ reactant feed can enter the reaction zone 208 *via* the inlet 204. The hydrocarbon reactant feed can be a mixture of butanes *(e.g.,* isobutane and n-butane) and enter reaction zone 208 *via* inlet 206. The reactant feed streams may include butanes, propane or trace C5s (*e.g*., hydrocarbons containing 5 carbon atoms). The reactant feeds may be premixed and provided at the same time. The reactant feed may be be provided in stages from H₂ rich to the desired H₂ to hydrocarbon ratio. The H₂ reactant feed and/or the hydrocarbon reactant feed may be be used to maintain a pressure in the reaction zone 208. The product stream can be removed from the reaction zone 208 *via* product outlet 210. The product stream can be sent to other processing units (*e.g*., separation units, isomerization units, and the like), stored, and/or transported.

The activated catalyst 212 may be a fresh catalyst that loses activity during the hydrogenolysis process forming a mixture of fresh and deactivated catalyst. At this point, the reactant feeds can be discontinued, and nitrogen added through nitrogen inlet 214. After the hydrogen and hydrocarbon reactant feed have been flushed from the system, a dilute oxygen stream can enter reactor 202 through oxygen inlet 214 and the activation process of the present invention can be implemented *(See,* Section A). For example, after the catalyst is treated with the dilute oxygen stream to form the oxidized catalyst, the oxygen feed can be discontinued and a nitrogen stream can enter the reactor *via* nitrogen inlet 214. After the catalyst and reactor have been flushed with nitrogen, the nitrogen stream can be discontinued, and the C2-C10 hydrocarbon stream can enter the reactor through hydrocarbon inlet 206 or a separate feed inlet and hydrogen can enter through hydrogen inlet 204. Contacting the catalyst with the stream comprising butane in the presence of H₂ using the activation process of the present invention forms the treated catalyst. After the treated catalyst is formed, the butane feed can be discontinued. The treated catalyst can then be contacted with hydrogen under the conditions of the activated catalyst process of the present invention to form the activated hydrogenolysis catalyst. After activation, the temperature of the catalyst and reactor can be adjusted to reaction temperatures and the hydrocarbon reactant feed can enter reactor 202 *via* hydrocarbon reactant feed 206 and the hydrogenolysis of hydrocarbons continues. The sequence of hydrogenolysis reaction / regeneration (activation) / hydrogenolysis reaction can continue as desired.

Reactor 202 can include one or more heating and/or cooling devices (e.g., insulation, electrical heaters, jacketed heat exchangers in the wall) or controllers (e.g., computers, flow valves, automated values, *etc.)* that can be used to control the reaction temperature and pressure of the reaction mixture. While only one reactor is shown, it should be understood that multiple reactors can be housed in one unit or a plurality of reactors housed in one heat transfer unit. A series of physically separated reactors with interstage cooling/heating devices, including heat exchangers, furnaces, fired heaters, and the like can be used.

The temperature, pressure, and WHSV can be varied depending on the reaction to be performed and is within the skill of a person performing the reaction *(e.g.,* an engineer or chemist). Temperatures can range from 240 °C to about 325 °C, 250 °C to 300 °C, 270 °C to 290 °C, or any value or range there between. Pressures can range from about 0.35 MPa to 1.4 MPa or 0.35, 0.40, 0.45, 0.50, 0.55, 0.60, 0.65, 0.70, 0.75, 0.8, 0.85, 0.9, 0.95, 1.0, 1.1, 1.2, 1.3, 1.4 or any range or value there between. A hydrocarbon (e.g., butane) WHSV can range from 1 to 10 hr⁻¹, or 1, 2, 3, 4, 5, 6, 7, 8, 9, 10 hr⁻¹ or any range or value there between.

The product stream can include methane, propane, ethane, and unreacted reactants. The products can be separated using known separation methodology. Produced methane can be used as a fuel for the system or can be reacted with steam to make hydrogen. Produced ethane can be sent to other processing units, for example sent to a steam cracker to produce ethylene. Produced propane can be sent to other processing units, for example, sent to a cracking unit together with ethane or used for on-purpose propylene production through propane dehydrogenation. Unreacted butane and/or hydrogen can be recycled to the reactor. The unreacted butane that is includes isobutane can be sent to a reverse-isomerization unit to increase the amount of n-butane in the unreacted feed stream.

Using the activated catalyst , the ethane selectivity can be at least 70%, 50 to 90 %, 70% to 80 %, or 70%, 75%, 80%, 85%, 90%, or any value or range there between. The ethane selectivity may be at least about 75%. Butane conversion can at least 50%, 50 to 95%, 70 to 90%, or at least 50%, 55%, 60%, 65%, 70%, 75%, 80%, 85% or 90%, or any value or range there between at a reaction temperature of 240 °C to 290 °C. Notably, hydrocarbon (e.g., butane) stability of an activated fresh catalyst was higher than the hydrocarbon stability of the original fresh catalyst under the same hydrogenolysis conditions.

### C. Catalyst

The catalyst can include a support and a catalytic bimetallic composition. The catalyst can be purchased (e.g., Zeolyst) or prepared using known catalyst preparation techniques. The catalyst can have a specific surface area of at least 100 m²/g, or 100 m²/g to 500 m²/g, or 100 m²/g, 150 m²/g, 200 m²/g, 250 m²/g, 300 m²/g, 350 m²/g, 400 m²/g, 450 m²/g, or 500 m²/g, or any value or range there between. The support can be alumina (Al₂O₃), titania (TiO₂), silica (SiO₂), a zeolite, or mixtures, or combinations thereof. Non-limiting examples of zeolites include ZSM-5, ZSM-11, Y, high-silica Y, USY, EU-1, EU-2, beta, L, ferrierite, CHA, SSZ-16, Nu-3, sigma-1, silicalite-1, and combinations thereof. The zeolite can be ZSM-5. The catalyst can include two noble metals. Non-limiting examples of noble metals include platinum (Pt), iridium (Ir), palladium (Pd), ruthenium (Rh), silver (Ag), gold (Au), an alloy thereof, or a combination thereof, preferably PtIr.

The catalyst can be made using impregnation methodology. Preferably, incipient wetness impregnation methodology can be used. Catalytic metal precursors can be dissolved in deionized water to form individual catalytic metal precursor solutions. Catalytic metal precursors can be obtained as a metal nitrate, a metal amine, a metal chloride, a metal coordination complex, a metal sulfate, a metal phosphate hydrate, metal complex, or any combination thereof. Examples of metal precursor compounds include hexachloroiridic acid and ammonium heptamolybdate. These metals or metal compounds can be purchased from any chemical supplier such as Sigma-Aldrich (St. Louis, Missouri, USA), Alfa-Aeaser (Ward Hill, Massachusetts, USA), and Strem Chemicals (Newburyport, Massachusetts, USA). The two solutions can be mixed to form a combined catalytic metal precursor solution or used separately. The catalytic metal precursor solutions or combined catalytic metal precursor solution can be added to a known quantity of support *(e.g.,* weighed alumina extrudates) and agitated for a period of time *(e.g.,* 2 to 24 hours) at ambient temperature (e.g., 20 °C to 35 °C) to form a catalytic metal precursor/support composition. The water can be removed by drying the catalytic metal precursor/support composition at a temperature of 80 °C to 100 °C, or about 90 °C. Once dried, the catalytic metal precursor/support composition can be calcined in air at 275 °C to 350 °C or 275 °C to 285 °C or any range or value there between. Calcination of the catalytic metal precursor/support composition forms the catalytic crystalline bimetallic composition and attaches the composition to the support.

### EXAMPLES

The present invention will be described in greater detail by way of specific examples.

### Example 1

### (Activation of a Fresh Hydrogenolysis Catalyst)

A fresh Pt(0.3 wt.%)-Ir(0.3 wt.%)/ZSM-5 catalyst (10 g, Zeolyst, USA) was pre-treated with the temperature protocol presented in Table 1 in hydrogen (Praxair, 99.999% purity) at a flowrate of 400 sccm at 110 psig (0.86 MPa) reactor pressure. The catalyst was then cooled down to reaction temperature (280 °C) in the same stream of hydrogen before introducing the feed. The catalyst activity was then studied at 280 °C, H₂/C₄H₁₀ molar ratio of 2.5, weight hourly space velocity w.r.t. C₄H₁₀ = 4 h⁻¹ and at 100 psig (0.79 MPa).

**Table 1**

| Segment | Temperature (°C) | Ramp rate (°C/min) | Hold time at end of segment (min) |
|---|---|---|---|
| 1 | RT a - 130 | 5 | 120 |
| 2 | 130-150 | 5 | 15 |
| 3 | 150 - 200 | 5 | 15 |
| 4 | 200 - 250 | 5 | 15 |
| 5 | 250 - 300 | 5 | 15 |
| 6 | 300 - 350 | 5 | 15 |
| 7 | 350 - 400 | 5 | 200 |

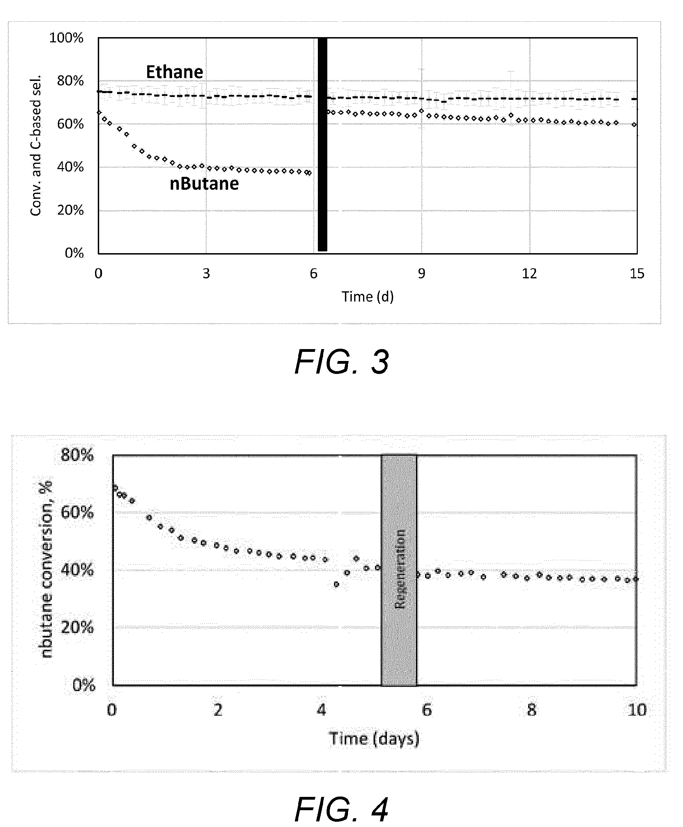

FIG. 3 shows the conversion of n-butane and the product selectivity to ethane as a function of time on stream. The conversion of n-butane dropped from about 65% to about 37% over a duration of 6 days due to catalyst deactivation. At this time, the reaction was stopped by zeroing the feed flowrates to the reactor. The catalyst was then purged with nitrogen before subjecting the catalyst to the regeneration protocol of the present invention as illustrated in FIG. 1. Upon re-introducing the feed, the conversion of n-butane increased to about 65% and also the rate of deactivation slowed relative the original untreated catalyst as shown in FIG. 4 over the next 6 to 7 days.

### Example 2

### (Regeneration/Activation of a Spent Hydrogenolysis Catalyst)

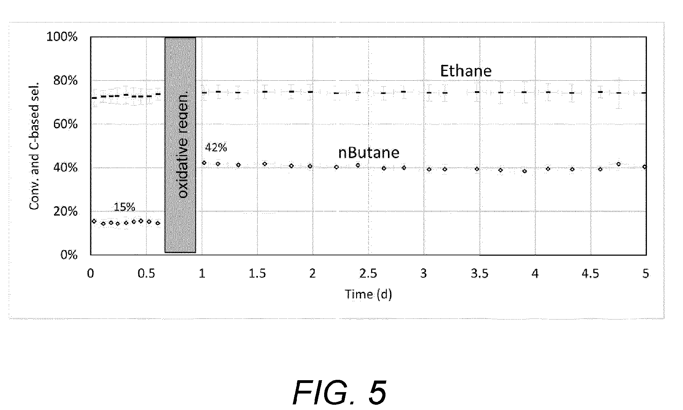

A Ir/Pt hydrogenolysis catalyst that had been used in a pilot plant butane hydrogenolysis operation for two months was obtained. The catalyst showed about 15% n-butane conversion before applying the regeneration process of the present invention. Post regeneration, the n-butane conversion increased to 42% and was stable for over four days as shown in FIG. 5. From the data, it was determined that the regeneration strategy was successful in recovering the activity of a spent catalyst.

### Example 3

### (Comparison of Inventive Treatment vs. Standard pretreatment)

Fresh Ir/Pt hydrogenolysis catalyst was divided into 2 portions. One portion of catalyst was treated using the activation treatment of the present invention using the treatment protocol listed in Table 2. The second catalyst portion was treated using standard catalyst pretreatment that involves only H₂ reduction at 400 °C. Reaction parameters for n-butane conversion of both studies were: hydrocarbon WHSV: 4h⁻¹, temperature: 280 °C, pressure: 100 psig (0.79 MPa), H₂/HC for inventive treated catalyst was 1.5 and H₂/HC for standard pretreatment catalyst was 2.5. The results are shown in FIG. 6. As shown, the catalyst having undergone the inventive protocol pretreatment had higher conversion and higher carbon-based selectivity (about 40% higher) than the catalyst having undergone a standard pretreatment protocol.

**Table 2**

| Segment | Temperature (°C) | Ramp rate (°C/min) | Hold time (min) | Treatment gas | Pressure |
|---|---|---|---|---|---|
| 1 | RTa → 130 | 1.5 | 120 | H₂ | 100 psig |
| 2 | 130 → 400 | 1.5 | 120 | H₂ | 100 psig |
| 3 | 400 → 350 | Cool down | 15 | N₂ | atmospheric |
| 4 | 350 | | 120 | 5% O₂/N₂ | atmospheric |
| 5 | 350 | | 15 | N₂ | atmospheric |
| 6 | 350 | | 2.5 | HC/H₂ | 100 psig |
| 7 | 350 | | 120 | H₂ | 100 psig |

| | | | | | |
|---|---|---|---|---|---|
| a) RT refers to room temperature In table 2 100 psig is equivalent to 0.79 MPa. | | | | | |

## Claims

1. A process for the activation of a hydrogenolysis catalyst, the process comprising:
(a) contacting an oxidized catalyst with a stream comprising butane in the presence of H₂ to form a treated catalyst; and thereafter
(b) contacting the treated catalyst with H₂ to form an activated hydrogenolysis catalyst.

2. The process of claim 1, wherein the stream comprising butane further comprises n-butane, isobutane or a combination thereof.

3. The process of any one of claims 1 to 2, wherein a weight ratio of H₂ to butane in step (a) is 0.5 to 20.

4. The process of any one of claims 1 to 3, wherein the weight ratio of butane to catalyst is 1 to 100.

5. The process of any one of claims 1 to 4, wherein conditions of step (a) comprise a temperature of 200 °C to 350 °C, preferably 275 °C to 325 °C, and a pressure of 0.5 MPa to 1.5 MPa, preferably 0.7 MPa to 0.8 MPa.

6. The process of any one of claims 1 to 5, wherein step (b) is performed for a time period longer than step (a).

7. The process of any one of claims 1 to 6, wherein conditions of step (b) comprise a temperature of 300 °C to 500 °C, preferably 300 °C to 400 °C, and a pressure of 0.5 MPa to 1.5 MPa, preferably, 0.7 MPa to 0.8 MPa.

8. The process of any one of claims 1 to 7, wherein the oxidized catalyst of step (a) is produced by contacting a fresh catalyst, a deactivated catalyst, or mixture thereof with an oxidizing stream comprising oxygen (O₂) and a diluent.

9. The process of claim 8, wherein contacting conditions comprises a temperature of 200 °C to 450 °C, preferably 400 °C or lower.

10. The process of any one of claims 8 to 9, wherein the oxidizing stream comprises 0.1 to 30 vol.% of O₂, preferably 5 to 20 vol.% O₂, or more preferably 8 to 12 vol. % O₂.

11. The process of any one of claims 1 to 10, wherein the catalyst comprises at least two noble metals on a support.

12. The process of claim 11, wherein the noble metal comprises platinum (Pt), iridium (Ir), palladium (Pd), ruthenium (Rh), silver (Ag), gold (Au), an alloy thereof, or a combination thereof, preferably PtIr.

13. The process of claims 11 to 12, wherein the support is a zeolite support, preferably ZSM5.

14. The process of claim 1, wherein the oxidized catalyst was made by impregnation method.

15. The process of claim 14, wherein the oxidized catalyst was made by incipient wet impregnation method.

## Patentansprüche

1. Verfahren zur Aktivierung eines Hydrogenolyse-Katalysators, wobei das Verfahren Folgendes umfasst:
(a) Inkontaktbringen eines oxidierten Katalysators mit einem Butan enthaltenden Strom unter Vorhandensein von H₂, um einen behandelten Katalysator zu bilden; und anschließend
(b) Inkontaktbringen des behandelten Katalysators mit H₂, um einen aktivierten Hydrogenolyse-Katalysator zu bilden.

2. Verfahren nach Anspruch 1, wobei der Butan enthaltende Strom ferner n-Butan, Isobutan oder eine Kombination davon enthält.

3. Verfahren nach einem der Ansprüche 1 bis 2, wobei das Gewichtsverhältnis von H₂ zu Butan in Schritt (a) 0,5 bis 20 beträgt.

4. Verfahren nach einem der Ansprüche 1 bis 3, wobei das Gewichtsverhältnis von Butan zum Katalysator 1 bis 100 beträgt.

5. Verfahren nach einem der Ansprüche 1 bis 4, wobei die Bedingungen von Schritt (a) eine Temperatur von 200 °C bis 350 °C umfassen, vorzugsweise 275 °C bis 325 °C, und einen Druck von 0,5 MPa bis 1,5 MPa, vorzugsweise 0,7 MPa bis 0,8 MPa.

6. Verfahren nach einem der Ansprüche 1 bis 5, wobei der Schritt (b) über einen längeren Zeitraum als Schritt (a) durchgeführt wird.

7. Verfahren nach einem der Ansprüche 1 bis 6, wobei die Bedingungen von Schritt (b) eine Temperatur von 300 °C bis 500 °C umfassen, vorzugsweise 300 °C bis 400 °C, und einen Druck von 0,5 MPa bis 1,5 MPa, vorzugsweise 0,7 MPa bis 0,8 MPa.

8. Verfahren nach einem der Ansprüche 1 bis 7, wobei der oxidierte Katalysator aus Schritt (a) hergestellt wird, indem ein frischer Katalysator, ein deaktivierter Katalysator oder eine Mischung davon mit einem oxidierenden Strom, der Sauerstoff (O₂) und ein Verdünnungsmittel umfasst, in Kontakt gebracht wird.

9. Verfahren nach Anspruch 8, wobei die Kontaktbedingungen eine Temperatur von 200 °C bis 450 °C umfassen, vorzugsweise 400 °C oder weniger.

10. Verfahren nach einem der Ansprüche 8 bis 9, wobei der oxidierende Strom 0,1 bis 30 Vol.-% O₂ umfasst, vorzugsweise 5 bis 20 Vol.-% O₂ oder noch bevorzugter 8 bis 12 Vol.-% O₂.

11. Verfahren nach einem der Ansprüche 1 bis 10, wobei der Katalysator mindestens zwei Edelmetalle auf einem Träger umfasst.

12. Verfahren nach Anspruch 11, wobei das Edelmetall Platin (Pt), Iridium (Ir), Palladium (Pd), Ruthenium (Rh), Silber (Ag), Gold (Au), eine Legierung davon oder eine Kombination davon, vorzugsweise PtIr, umfasst.

13. Verfahren nach den Ansprüchen 11 bis 12, wobei der Träger ein Zeolithträger, vorzugsweise ZSM5, ist.

14. Verfahren nach Anspruch 1, wobei der oxidierte Katalysator durch ein Imprägnierungsverfahren hergestellt wurde.

15. Verfahren nach Anspruch 14, wobei der oxidierte Katalysator durch ein Imprägnierungsverfahren nach der incipient-wetness-Methode hergestellt wurde.

## Revendications

1. Procédé pour l'activation d'un catalyseur d'hydrogénolyse, le procédé comprenant :
(a) la mise en contact d'un catalyseur oxydé avec un flux comprenant du butane en présence de H₂ pour former un catalyseur traité ; et par la suite
(b) la mise en contact du catalyseur traité avec H₂ pour former un catalyseur d'hydrogénolyse activé.

2. Procédé selon la revendication 1, dans lequel le flux comprenant du butane comprend en outre du n-butane, de l'isobutane ou une combinaison de ceux-ci.

3. Procédé selon l'une quelconque des revendications 1 et 2, dans lequel un rapport pondéral entre H₂ et le butane dans l'étape (a) est de 0,5 à 20.

4. Procédé selon l'une quelconque des revendications 1 à 3, dans lequel le rapport pondéral entre le butane et le catalyseur est de 1 à 100.

5. Procédé selon l'une quelconque des revendications 1 à 4, dans lequel les conditions de l'étape (a) comprennent une température de 200 °C à 350 °C, de préférence de 275 °C à 325 °C, et une pression de 0,5 MPa à 1,5 MPa, de préférence de 0,7 MPa à 0,8 MPa.

6. Procédé selon l'une quelconque des revendications 1 à 5, dans lequel l'étape (b) est réalisée pendant une période plus longue que l'étape (a).

7. Procédé selon l'une quelconque des revendications 1 à 6, dans lequel les conditions de l'étape (b) comprennent une température de 300 °C à 500 °C, de préférence de 300 °C à 400 °C, et une pression de 0,5 MPa à 1,5 MPa, de préférence de 0,7 MPa à 0,8 MPa.

8. Procédé selon l'une quelconque des revendications 1 à 7, dans lequel le catalyseur oxydé de l'étape (a) est produit par mise en contact d'un catalyseur frais, d'un catalyseur désactivé, ou d'un mélange de ceux-ci avec un flux oxydant comprenant de l'oxygène (O₂) et un diluant.

9. Procédé selon la revendication 8, dans lequel les conditions de mise en contact comprennent une température de 200 °C à 450 °C, de préférence inférieure ou égale à 400 °C.

10. Procédé selon l'une quelconque des revendications 8 et 9, dans lequel le flux oxydant comprend 0,1 à 30 % en volume d'O₂, de préférence 5 à 20 % en volume d'O₂, ou plus préférablement 8 à 12 % en volume d'O₂.

11. Procédé selon l'une quelconque des revendications 1 à 10, dans lequel le catalyseur comprend au moins deux métaux nobles sur un support.

12. Procédé selon la revendication 11, dans lequel le métal noble comprend du platine (Pt), de l'iridium (Ir), du palladium (Pd), du ruthénium (Rh), de l'argent (Ag), de l'or (Au), un alliage de ceux-ci, ou une combinaison de ceux-ci, de préférence Ptlr.

13. Procédé selon les revendications 11 et 12, dans lequel le support est un support zéolitique, de préférence ZSM5.

14. Procédé selon la revendication 1, dans lequel le catalyseur oxydé a été fabriqué par une méthode d'imprégnation.

15. Procédé selon la revendication 14, dans lequel le catalyseur oxydé a été fabriqué par une méthode d'imprégnation à humidité naissante.
